# FASCICULE DE BREVET EUROPEEN

(11) **EP 1 913 130 B1**
(45) Date de publication et mention de la délivrance du brevet: **23.12.2015**
(21) Numéro de dépôt: 06794234.2
(22) Date de dépôt: 27.07.2006
(51) Int. Cl.: C12N 5/071, A61K 35/44, A61K 47/48

(54) **SYSTEME MARQUEUR CELLULAIRE/LIGAND, OU LE MARQUEUR EST DU TYPE EPH, MATERIAU CELLULAIRE COMPRENANT CE SYSTEME, PROCEDE DE PREPARATION ET UTILISATION PROANGIOGENIQUE**
ZELL-/LIGANDENMARKIERUNGSSYSTEM MIT MARKER VOM EPH-TYP, DAS SYSTEM ENTHALTENDES ZELLMATERIAL, VERFAHREN ZUR HERSTELLUNG DAVON UND PROANGIOGENE VERWENDUNG
CELL/LIGAND MARKING SYSTEM, WHEREIN THE MARKER IS OF EPH TYPE, CELL MATERIAL COMPRISING SAID SYSTEM, METHOD FOR PREPARING SAME AND PROANGIOGENETIC USE

(30) Priorité: 27.07.2005 FR 0508029
(43) Date de publication de la demande: 23.04.2008
(73) Titulaire: INSTITUT DES VAISSEAUX ET DU SANG, 75475 Paris Cedex 10 (FR); Université Paris Diderot - Paris 7, 75205 Paris Cedex 13 (FR)
(72) Inventeur: FOUBERT, Philippe, F-94220 Charenton le Pont (FR); SILVESTRE, Jean-Sébastien, F-75012 Paris (FR); LE RICOUSSE-ROUSSANNE, Sophie, F-94500 Champigny-sur-Marne (FR)
(74) Mandataire: Bertrand, Didier
(86) Numéro de dépôt international: PCT/FR2006/001837
(87) Numéro de publication internationale: WO 2007/012764

(56) Documents cités:
- WO-A-98/19712
- US-A- 5 541 103
- US-B1- 6 864 227
- LE RICOUSSE-ROUSSANNE SOPHIE ET AL: "Ex vivo differentiated endothelial and smooth muscle cells from human cord blood progenitors home to the angiogenic tumor vasculature" CARDIOVASCULAR RESEARCH, vol. 62, no. 1, 1 avril 2004 (2004-04-01), pages 176-184, XP002385798 ISSN: 0008-6363
- MAEKAWA HIROMITSU ET AL: "Ephrin-B2 induces migration of endothelial cells through the phosphatidylinositol-3 kinase pathway and promotes angiogenesis in adult vasculature." ARTERIOSCLEROSIS THROMBOSIS AND VASCULAR BIOLOGY, vol. 23, no. 11, novembre 2003 (2003-11), pages 2008-2014, XP002385797 ISSN: 1079-5642
- ATSUHIKO KAWAMOTO ET AL: "Therapeutic potential of ex vivo expanded endothelial progenitor cells for myocardial ischemia" CIRCULATION, vol. 103, 2001, pages 634-637, XP002268640
- SHIN DONGHUN ET AL: "Expression of ephrinB2 identifies a stable genetic difference between arterial and venous vascular smooth muscle as well as endothelial cells, and marks subsets of microvessels at sites of adult neovascularization" DEVELOPMENTAL BIOLOGY, vol. 230, no. 2, 15 février 2001 (2001-02-15), pages 139-150, XP002385076 ISSN: 0012-1606
- WANG ZHENGYU ET AL: "Ephrin receptor, EphB4, regulates ES cell differentiation of primitive mammalian hemangioblasts, blood, cardiomyocytes, and blood vessels." BLOOD, vol. 103, no. 1, 1 janvier 2004 (2004-01-01), pages 100-109, XP002385074 ISSN: 0006-4971 cité dans la demande
- DZAU VICTOR J ET AL: "Therapeutic potential of endothelial progenitor cells in cardiovascular diseases" HYPERTENSION (BALTIMORE), vol. 46, no. 1, juillet 2005 (2005-07), pages 7-18, XP002422113 epub. 13 juin 2005 ISSN: 0194-911X
- LUTTUN A ET AL: "Vascular progenitors: From biology to treatment" TRENDS IN CARDIOVASCULAR MEDICINE 2002 UNITED STATES, vol. 12, no. 2, 2002, pages 88-96, XP002385799 ISSN: 1050-1738
- CARMELIET PETER: "Angiogenesis in health and disease." NATURE MEDICINE, vol. 9, no. 6, juin 2003 (2003-06), pages 653-660, XP002422112 ISSN: 1078-8956
- NOREN N K ET AL: "Interplay between EphB4 on tumor cells and vascular ephrin-B2 regulates tumor growth" PROCEEDINGS OF THE NATIONAL ACADEMY OF SCIENCES OF USA, NATIONAL ACADEMY OF SCIENCE, WASHINGTON, DC, US, vol. 101, no. 15, 13 avril 2004 (2004-04-13), pages 5583-5588, XP002992943 ISSN: 0027-8424 cité dans la demande
- WANG H U ET AL: "Molecular distinction and angiogenic interaction between embryonic arteries and veins revealed by ephrin - B2 and its receptor Eph - B4 [see comments]" CELL, CELL PRESS, CAMBRIDGE, NA, US, vol. 93, no. 5, 29 mai 1998 (1998-05-29), pages 741-753, XP002120944 ISSN: 0092-8674 cité dans la demande

## Description

### Domaine de l'invention

La présente invention a trait à une nouvelle solution technique mettant en oeuvre un système comprenant un marqueur cellulaire et un ligand spécifique dudit marqueur, où ledit marqueur est choisi parmi l'ensemble constitué par les Eph, notamment le marqueur EphB4. Elle concerne également le matériau cellulaire comprenant ce système ainsi que son procédé de préparation et son utilisation en thérapeutique en tant qu'agent proangiogénique.

### Art antérieur

On sait que les membres de la famille des éphrines et de leurs récepteurs Eph à tyrosine kinase, qui ont été dans un premier temps mis en évidence dans le système nerveux pour le guidage des neurones, sont des facteurs intervenant dans l'angiogenèse. De nombreuses isoformes pour les récepteurs Eph et leurs ligands éphrines ont été décrites avec des spécificités d'expression tissulaire. Chez les vertébrés, on connaît au moins 16 récepteurs Eph, à savoir : 10 récepteurs EphA (EphA1 à EphA10) et 6 récepteurs EphB (EphB1 à EphB6), d'une part, et au moins 9 ligands éphrine, à savoir : éphrine-A1 à éphrine-A6 et éphrine-B1 à éphrine-B3, d'autre part. A l'origine, la subdivision en deux classes, EphA et EphB, était fondée sur l'homologie de la séquence de leur domaine extracellulaire, mais cette subdivision correspond aussi à la liaison préférentielle de leurs ligands, les ligands éphrine-A étant en général liés à la membrane par un glycosylphosphatidilinositol (GPI) et les ligands éphrine-B étant transmembranaires avec un domaine intracytoplasmique possédant undomaine de liaison PDZ.

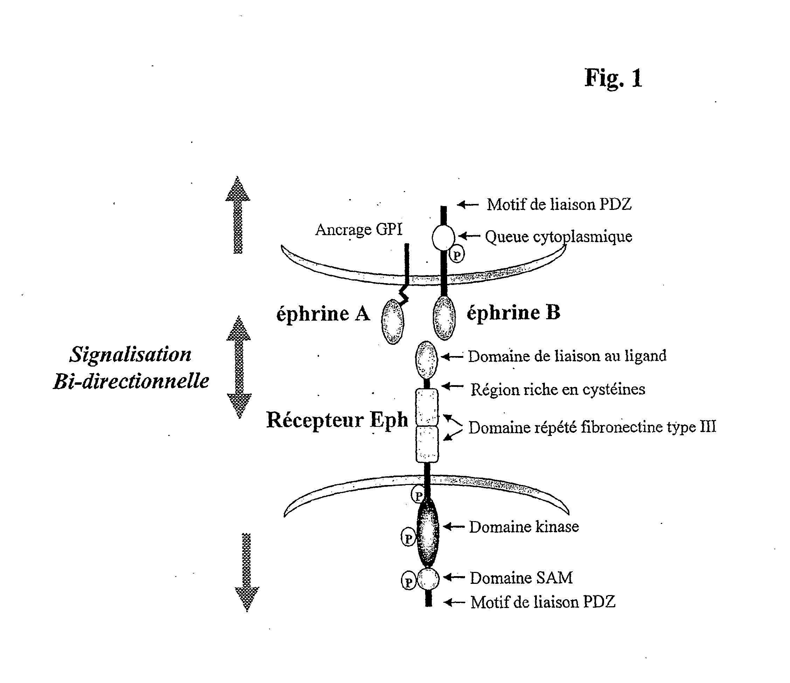

Comme représenté à la figure 1 ci-après :
- le récepteur Eph comporte dans le domaine extracellulaire, des unités répétées de fibronectine de type III (en anglais :
   "fibronectin type III repeats"), une région riche en cystéine ("cystein rich region") et un domaine de liaison pour un ligand spécifique ("ligand-binding domain") ; il présente en outre une prolongation dans le domaine intracellulaire comportant un domaine à activité tyrosine kinase, un motif α-stérile ("SAM") et
   un motif de liaison PDZ ("PDZ-binding motif");

- le ligand éphrine-A est accroché à la membrane par un GPI ("GPI anchor") ; et
- le ligand transmembranaire éphrine-B présente une prolongation intracellulaire comportant une queue cytoplasmique (en anglais : "cytoplasmic tail") et un motif de liaison PDZ.

Eu égard à l'interaction entre le récepteur Eph (notamment EphB4), à tyrosine kinase, et son ligand (notamment éphrine-B2), protéine transmembranaire, on a une "signalisation bidirectionnnelle" ("bidirectionnal signaling") qui comprend une signalisation induite par le récepteur Eph dans la cellule exprimant Eph sur sa surface ("forward signaling"), et une signalisation "reverse" induite par le ligand éphrine dans la cellule exprimant éphrine à sa surface ("reverse signaling"). Cette signalisation bidirectionnelle joue un rôle lors des contacts cellules/cellules, de la migration, et de l'adhésion des cellules.

On connaît de l'article de Wang H. U. et al., "Molecular distinction and angiogenic interaction between embryonic arteries and veins revealed by ephrin-B2 and its receptor EphB4", Cell, 1998; 93: 741-753*,* la spécificité d'expression artério-veineuse du couple EphB4/éphrine-B2, EphB4 étant uniquement exprimé par les veines, et éphrine-B2 étant exprimé par les artères.

Les récepteurs EphB et leurs ligands éphrine-B sont exprimés particulièrement durant le développement embryonnaire. Selon Wang Z. et al., "Ephrin receptor, EphB4, regulates ES cell differentiation of primitive mamalian hemangioblasts, blood, cardiomyocytes and blood vessels", Blood, 2004; 103: 100-109*,* EphB4 semble intervenir lors de la différenciation de cellules embryonnaires (ES) de souris en cellules endothéliales.

On sait en outre que certains récepteurs Eph, notamment EphB4, sont surexprimés dans les tumeurs. Cette observation suggère que ces récepteurs Eph jouent un rôle dans la progression tumorale. L'article de Noren N. K. et al., "Interplay between EphB4 on tumor cells and vascular ephrin-B2 regulates tumor growth", Proc. Natl. Acad. Sci. USA, 101: 5583-5588*,* démontre que l'activation de éphrine-B2 par EphB4 (signalisation reverse) stimule la croissance tumorale.

En revanche, ce qui est observé par la littérature antérieure c'est (i) une augmentation de l'expression d'éphrine-B2 au cours de la tumorigenèse, et subsidiairement (ii) une inquiétude, du fait de cette augmentation, qu'éphrine-B2 serait susceptible d'induire des tumeurs. Ceci explique que plusieurs publications proposent d'utiliser la détection d'éphrine-B2 comme marqueur tumoral, d'une part, ou d'inhiber éphrine-B2, d'autre part. Voir en particulier (i) WO 02/058538 A, qui préconise la détection du ligand éphrine-B2 en tant que marqueur de la vasculature tumorale et décrit une technique permettant de visualiser ladite vasculature tumorale, (ii) US 2003/0207447 A, qui propose de faire appel à un inhibiteur de Eph, notamment EphB4, ou à un inhibiteur d'éphrine, notamment d'éphrine-B2, pour limiter l'angiogenèse en empêchant l'interaction entre le récepteur et le ligand, (iii) US 6864227 A et WO 03/102144 A, qui recommandent d'utiliser un anticorps, soit un anti(Eph) [notamment un anticorps anti(EphB4)], soit un anti(éphrine) [notamment anti(éphrine-B2)], pour réduire ou moduler l'angiogenèse.

En bref, les documents de brevet publiés relatifs au couple Eph/éphrine concernent des méthodes ayant trait notamment à :
- la signalisation des éphrine-B (signalisation reverse) par des protéines se liant au domaine PDZ, voir WO 02/079382 A et WO 00/031124 ;
- la modulation de l'expression d'éphrine-B2 ou d'EphB4, voir notamment US 2004/0110150 A, WO 04/006846 A et WO 04/080418 ;
- l'inhibition de l'interaction éphrine-B2/EphB4 en vue de traiter les cancers et les maladies associées à l'angiogenèse, voir notamment le document WO 04/080418 A précité, et WO 04/069264 A ; et
- l'utilisation d'éphrine-B2 en tant que marqueur de la vasculature tumorale, voir notamment le document WO 02/058538 A précité.

En outre, l'article de MAEKAWA HIROMITSU et Al « Ephrin-B2 induces migration of endothélial cells through the phosphatidylinositol-3 kinase pathway and promotes angiogenesis in adult vasculature », Arteriorclerosis thrombosis and vascular biology, 2003, 23 : 2008-2014*,* montre que le ligand éphrine-B2, couplé à la protéine Fc (i.e. le fragment Fc d'un anticorps), induit la migration de cellules endothéliales, les HUVECs.

Enfin, on connaît de US 6610534 B un adénovirus ayant la séquence codante de la sphingosine kinase et une séquence codante d'une protéine angiogénique. L'objectif de ce brevet est l'expression de la sphingosine kinase et de la protéine angiogénique, qui peut-être éphrine-B2, après injection locale dudit adénovirus. Par ailleurs, on connaît de la publication US 2005/0049194 A une méthode pour diminuer la prolifération anormale de cellules souches hématopoïétiques, qui comprend l'administration par injection d'un inhibiteur hydrosoluble d'éphrine-B2.

En outre, Kawamoto et al. (Circulation, vol. 103, 2001, pages 634-637) divulgue le potentiel thérapeutique d'EPC pour la néovascularisation du myocarde. Le document de brevet WO 98/19712A décrit un procédé de régulation de l'angiogenèse utilisant des précurseurs de cellules endothéliales.

### But de l'invention

Selon l'invention on se propose de fournir une nouvelle solution, non décrite ni suggérée par l'art antérieur précité, au problème technique de la stimulation de l'angiogenèse. Plus précisément, on se propose ici de fournir une nouvelle solution technique mettant en oeuvre un système marqueur cellulaire/ligand spécifique dudit marqueur, qui est appliqué à des cellules particulières, à savoir des précurseurs de cellules endothéliales, pour résoudre le problème de la stimulation angiogénique. Le but poursuivi est d'améliorer les thérapies cellulaires antérieurement connues en activant les cellules avant leur injection, pour avoir une efficacité supérieure des processus de revascularisation, notamment la revascularisation post-ischémique.

### Objet de l'invention

La nouvelle solution selon l'invention met en oeuvre un système marqueur cellulaire/ligand, où le dit marqueur est associé à une cellule précurseur de cellules endothéliales. Dans ce système, le marqueur est un récepteur Eph, et le ligand spécifique d'Eph est un ligand éphrine.

Selon un premier aspect de l'invention, on fournit un nouveau système proangiogénique du type marqueur cellulaire/ligand spécifique, ledit système, dans lequel le marqueur cellulaire est présent sur la membrane extérieure de la cellule, étant caractérisé en ce qu'il comprend :
(a) un précurseur de cellule endothéliale (EPC) comportant un marqueur cellulaire choisi parmi l'ensemble constitué par les marqueurs cellulaire EphB, notamment EphB4 ou EphB1, et
(b) un matériau protéinique de structure :

   L-K (I)

   qui est constitué d'un ligand (L) choisi parmi les ligands éphrine-B ou un fragment peptidique de ceux-ci ayant la même activité biologique, et est associé ou fusionné avec une protéine de liaison (K) choisie parmi des fragments Fc d'anticorps A,
   ledit précurseur de cellule endothéliale (a) et ledit matériau protéinique (b) sont conditionnés séparément l'un de l'autre,
   et en ce que l'incubation dudit précurseur de cellule endothéliale (a) et dudit matériau protéinique (b) fournit un matériau cellulaire de structure :

   EPC-EphB-L-K (II)
pour stimuler l'angiogenèse.

En d'autres termes, pour stimuler l'angiogenèse on active les cellules EPCs comportant le marqueur Eph par le ligand L spécifique appartenant à la famille des éphrines. Le ligand L peut également être constitué d'un fragment peptidique d'une éphrine par exemple de éphrine-B2 qui aurait alors la même activité biologique.

Selon un second aspect de l'invention, on fournit un matériau cellulaire capable de stimuler l'angiogenèse, caractérisé en ce qu'il a pour structure :

EPC-Eph-L-K (II)

où ledit ligand L est associé à ou fusionné avec une protéine de liaison K.

Ce matériau cellulaire est susceptible (i) de se présenter sous la forme d'une culture cellulaire substantiellement purifiée ou sous la forme d'une culture cellulaire en association avec d'autres cellules précurseurs, notamment des cellules mononucléées, et (ii) d'être; le cas échéant, congelé.

Selon un troisième aspect de l'invention, on fournit un procédé pour la préparation dudit matériau cellulaire, ledit procédé étant caractérisé en ce qu'il comprend les étapes consistant à :
- faire appel à des EPCs exprimant sur leur membrane extérieure un marqueur de la famille des Eph, notamment EphB4 ou EphB1, et
- mettre en contact *in vitro* lesdits EPCs avec un matériau protéinique L-K où L est un ligand spécifique dudit marqueur et est choisi parmi les ligands Ephrine-B ou un fragment peptidique de ceux-ci ayant la même activité biologique; et K est une protéine de liaison associée ou fusionnée à L choisie parmi des fragments Fc d'anticorps A.

On préconise également un médicament utilisable pour reconstituer les vaisseaux lésés, caractérisé en ce qu'il comprend, en association avec un excipient physiologiquement acceptable, une quantité thérapeutiquement acceptable du matériau cellulaire selon l'invention, notamment en tant qu'ingrédient actif proangiogénique.

On fournit enfin, selon un autre aspect de l'invention, une nouvelle utilisation dudit matériau cellulaire, ladite utilisation étant caractérisée en ce que l'on fait appel audit matériau cellulaire, en tant qu'ingrédient actif proangiogénique, en association avec un excipient physiologiquement acceptable, pour la préparation d'une composition pour usage thérapeutique dans le traitement des insuffisances vasculaires, notamment dans la revascularisation des tissus ischémiés, cardiaques, cérébraux ou périphériques.

### Brève description des dessins

Dans les dessins annexés,
- la figure 1, présentée en détail plus haut, illustre l'état antérieur des connaissances et représente schématiquement les récepteurs à tyrosine kinase, Eph, et leurs ligands du type éphrine-A ou du type éphrine-B ; et
- les figures 2 à 4 montrent graphiquement les propriétés proangiogéniques, eu égard aux essais qui ont été entrepris, du matériau cellulaire selon l'invention.

### Description détaillée de l'invention

On connaît, notamment de l'article de Pasquale E. B., Curr. Opin. Cell Biol., 1997; 9(5):608*,* les spécificités des composants du système récepteur Eph/ligand éphrine Par commodité le tableau I, donné plus loin et établi d'après cet article (comme indiqué dans US 6579683 A), fournit ces spécificités pour certains couples, les ligands étant placés dans la seconde colonne par affinité décroissante vis-à-vis de leurs récepteurs consignés dans la première colonne.

Dans le système marqueur cellulaire/ligand spécifique selon l'invention, on peut concevoir que les deux composants du couple peuvent se présenter chacun sous la forme d'une séquence d'aminoacides ou, le cas échéant, sous la forme d'une séquence d'acides nucléiques. Il est cependant nettement plutôt préférable de disposer au départ d'un récepteur Eph exprimé sur la membrane de EPC sous la forme d'une séquence d'aminoacides. De même, le ligand éphrine sera très avantageusement utilisé sous la forme d'une séquence d'aminoacides ou bien d'un fragment peptidique éphrine. En effet, c'est sous la forme d'aminoacides que Eph et éphrine se lient, l'interaction protéine/protéine étant nécessaire pour observer l'activité proangiogénique. En conséquence, dans ce qui suit, Eph et éphrine interviennent chacun exclusivement sous la forme protéinique d'une séquence d'aminoacides.

En tant que marqueur Eph, on peut utiliser ici un EphA ou un EphB. Cependant, on préfère plutôt selon l'invention que le marqueur Eph soit un EphB, dès lors que les marqueurs EphB sont impliqués de façon prépondérante dans l'angiogenèse, alors que (dans l'état actuel des connaissances) les marqueurs EphA semblent intervenir principalement au niveau du système nerveux.

Selon l'invention, de façon avantageuse le marqueur EphB sera EphB4 ou EphB1 ; parmi les marqueurs EphB, EphB4 sera préféré à EphB1.

Parmi les ligands éphrine, on préfère selon l'invention les ligands éphrine-B, notamment éphrine-B2 ou éphrine-B1. En outre, un variant de éphrine-B2, par exemple, qui correspond à un fragment peptidique de éphrine-B2 présentant la même activité biologique peut également être approprié.

En conséquence, dans le système selon l'invention on utilisera avantageusement le marqueur EphB4 ou EphB1, d'une part, et le ligand éphrine-B2 ou éphrine-B1, d'autre part, le couple Eph/éphrine plus particulièrement préféré selon l'invention étant EphB4/éphrine-B2.

Pour que le ligand L puisse activer EPC-Eph, il est important, dans la majorité des cas, qu'il soit associé à ou fusionné avec une protéine de liaison K sous la forme d'un matériau protéinique de structure :

L-K (I)

la protéine éphrine-B2 recombinante étant, à la connaissance du Déposant, une substance susceptible d'activer EPC-Eph seule sans l'apport de ladite protéine de liaison K.

Parmi les protéines de liaison K, qui conviennent selon l'invention, on peut notamment cité les nombreux fragments Fc d'anticorps (par exemples ceux obtenus par clivage d'anticorps par la pepsine, la papaïne ou toute autre substance appropriée). Par commodité, on recommande ici un fragment Fc, aisément disponible sur le marché en tant que sous produit de la préparation d'anticorps tels que Fab' et F(ab)².

En variante et compte tenu de ce qui précède, ledit matériau protéinique L-K est susceptible d'être remplacé par la protéine éphrine-B2 recombinante, de préférence humaine.

Les EPCs, qui conviennent selon l'invention, sont des cellules qui comportent un marqueur cellulaire Eph (notamment un EphB, de préférence EphB1 ou mieux EphB4) exprimé au niveau de leur membrane extérieure. De telles cellules EPCs sont obtenues à partir de cellules mononucléées ou de cellules exprimant CD34 ou CD133, qui proviennent de la moelle osseuse, du sang périphérique ou mieux du sang de cordon ombilical.

Les cellules mononucléées sont produites au niveau de la moelle osseuse, où elles se trouvent à une concentration importante, passent dans la circulation sanguine et se retrouvent dans le sang de cordon et dans le sang périphérique. Elles constituent un matériau de choix , en ce sens que parmi leur ensemble, un nombre important de cellules possède le matériel génétique requis pour (i) exprimer le marqueur Eph (et plus particulièrement le marqueur EphB4), ou (ii) comporter sur leur membrane extérieure ledit marqueur déjà exprimé. Les cellules mononucléées préférées selon l'invention sont celles qui sont CD34⁺ ou CD133⁺, car elles fournissent après différenciation une quantité relativement importante de EPCs qui expriment ou comportent substantiellement le marqueur EphB4.

La concentration des EPCs produites par différenciation, qui sont contenues dans la population de cellules mononucléées, varie selon l'origine des cellules. Ladite concentration est à peu près équivalente dans la moelle osseuse et le sang de cordon ombilical. En revanche, elle est plus faible dans le sang périphérique.

Le matériau cellulaire selon l'invention, qui est représenté par la structure II ci-dessus, dans laquelle le matériau protéinique L-K est susceptible d'être remplacé par la protéine éphrine-B2 recombinante, est constitué d'une ou plusieurs cellules, possédant chacune sur sa membrane extérieure un marqueur Eph, notamment EphB4 ou EphB 1, lié à son ligand spécifique. En variante, ledit matériau cellulaire peut être constitué d'une culture contenant plusieurs cellules de structure II en association, le cas échéant, avec d'autres cellules non activées par ledit matériau protéinique ; une telle culture peut donc être un mélange cellulaire de EPCs activées, de EPCs non activées et de cellules mononucléées.

Selon un mode particulier de l'invention, ledit matériau cellulaire est obtenu par incubation
(a) d'un précurseur de cellule endothéliale (EPC) comportant un marqueur cellulaire choisi parmi l'ensemble constitué par les EphB, notamment EphB4 ou EphB1, avec
(b) un matériau protéinique de structure :

   L-K (I)

   qui est constitué d'un ligand (L) spécifique dudit marqueur, et est associé ou fusionné avec une protéine de liaison (K), choisie parmi un fragment Fc d'anticorps A,
avant d'être amené à son site d'administration.

Selon un mode avantageux de réalisation, l'incubation est effectuée *in vitro* pendant une durée de 10 à 60 minutes, notamment pendant 30 minutes, cette durée d'incubation se situant juste avant l'administration du matériau cellulaire.

En variante, ledit matériau cellulaire peut se trouver sous la forme d'une culture cellulaire qui est purifiée ou qui est un mélange de cellules contenant (i) des cellules EPCs activées par L-K ou la protéine éphrine-B2 recombinante, et (ii) des précurseurs non activé, par exemple des cellules mononucléées et/ou EPCs, qui ne sont pas activées par L-K ou ladite protéine éphrine-B2 recombinante. De plus, le matériau cellulaire peut être conservé à l'état congelé.

Le matériau cellulaire préféré selon l'invention, eu égard à ce qui a été indiqué plus haut, est de structure II où ledit marqueur est EphB4 ou EphB1, et où ledit ligand est éphrine-B2 ou éphrine-B1.

Suivant un mode préféré de mise en oeuvre, le procédé de l'invention pour préparer ledit matériau cellulaire, comprend les étapes suivantes :
(1°) faire appel à des cellules mononucléées provenant de la moelle osseuse, du sang périphérique ou du sang de cordon ombilical, et isoler les cellules mononucléées CD34⁺ et/ou CD133⁺.
(2°) différencier lesdites cellules de l'étape précédente pour obtenir des EPCs pourvues du marqueur EphB, et
(3°) activer *in vitro* les EPCs, ainsi obtenues à l'étape (2°), par fixation d'éphrine-B sur EphB.

Ledit procédé comprend, une étape supplémentaire entre les étapes (1°) et (2°), à savoir:
(1a°) isoler les cellules mononucléées CD34⁺ et/ou CD133⁺.

L'étape (1a°) implique un processus ternaire (1°) + (1a°) + (2°). Toutefois, la mise en oeuvre de ladite étape (1a°) augmente les coûts de production par rapport au processus binaire (1°) + (2°). En pratique, comme les cellules EPCs non activées servent à la dilution des cellules EPCs activées dans le milieu cellulaire les contenant, sans gêner leur action, le processus binaire est à l'heure actuelle manifestement plus rentable que le processus ternaire.

Selon le procédé de l'invention, ledit ligand L est associé à ou fusionné avec une protéine de liaison K pour fournir un matériau cellulaire de structure :

EPC-Eph-L-K (II)

où avantageusement ledit marqueur Eph est EphB4 ou EphB1, et ledit ligand L est éphrine-B2 ou éphrine-B1.

En tant que médicament, le matériau cellulaire selon l'invention est utilisable pour traiter notamment les insuffisances vasculaires, en particulier dans la revascularisation des tissus ischémiés cardiaques, cérébraux ou périphériques.

Le matériau cellulaire selon l'invention peut être conditionné sous forme de dose unitaire, chaque dose contenant le matériau de structure II. En variante, on peut prévoir un conditionnement selon lequel les composants dudit matériau II, à savoir EPC-Eph et le matériau protéinique L-K, ne sont pas en contact ; l'incubation selon le mécanisme réactionnel :

EPC-Eph + K-L → EPC-Eph-K-L

étant effectuée avant administration, notamment pendant les 10 à 60 minutes (de préférence pendant les 20 à 30 minutes) qui précèdent cette administration.

Dans ce cas, on préconise une composition médicamenteuse, qui est caractérisée en ce qu'elle comprend, une quantité thérapeutiquement acceptable des deux composants du système proangiogénique selon l'invention, qui sont conditionnés séparément, chacun dans un excipient physiologiquement acceptable, les deux dits composants étant incubés avant administration pour donner un matériau cellulaire de structure II.

Comme indiqué plus haut, le matériau cellulaire selon l'invention est utile pour régénérer les tissus vasculaires qui ont été endommagés notamment au niveau cardiaque, cérébral ou périphérique. Il convient en particulier pour la préparation d'une composition destinée au traitement de l'artérite, de l'insuffisance vasculaire coronarienne, ou cardiaque, et de l'insuffisance vasculaire cérébrale. Sur un modèle animal, il a fourni de bons résultats dans le traitement de l'ischémie dite critique des membres inférieurs, d'une part, et l'assurance d'une guérison permettant d'éviter l'amputation.

Le matériau cellulaire selon l'invention peut être administré chez le mammifère et en particulier chez l'homme selon un mode connu en soi. Par exemple ledit matériau cellulaire est susceptible d'être (i) injecté au niveau ou au voisinage de la lésion vasculaire, (ii) injecté dans le sang par voie IV, ou encore (iii) amené au site de la lésion au moyen d'un vecteur approprié. En variante, on peut administrer séparément par voie injectable (notamment par voie IV) les cellules EPC-Eph, d'une part, et le matériau protéinique lié à un vecteur connu dans le domaine de la thérapie génique, d'autre part.

Chez l'homme adulte, on peut administrer par injection IV des cellules de structure II contenues, le cas échéant, dans un mélange cellulaire de EPCs non activées. Un tel mélange cellulaire peut comporter un total d'environ 10⁵ à 10⁹ cellules par injection.

D'autres avantages et caractéristiques de l'invention seront mieux compris à la lecture qui va suivre d'exemples de préparation et d'essais pharmacologiques. Bien entendu, l'ensemble de ces éléments n'est pas limitatif mais est donné à titre d'illustration, le matériau cellulaire utilisé étant : EPC-EphB4-éphrine-B2-Fc.

### Exemple 1

### Obtention de EPC-EphB4

(***A***) On prélève des échantillons (de 30 à 50 ml chacun) de sang de cordon ombilical humain que l'on place dans des tubes stériles contenant une solution anticoagulante d'héparine sodique. On isole, par centrifugation de gradient de densité au moyen de Pancoll (1,077 g/ml, produit commercialisé par la société dite DOMINIQUE DUTSCHER S.A, Brumath, France), les cellules mononucléées du sang de cordon ombilical. Les cellules mononucléées sont ensuite séparées des cellules adhérentes par culture sur des boites en plastique pendant 24 heures à 37°C. On obtient un mélange cellulaire contenant des cellules mononucléées exprimant le marqueur EphB4 et des cellules mononucléées n'exprimant pas ledit marqueur.
***(B)*** Le mélange cellulaire, obtenu à l'issue de l'exemple 1(A), est placé dans les puits d'une plaque à 6 puits revêtus de collagène de type I (produit commercialisé par la société dite SIGMA-ALDRICH, Saint-Quentin, France) dans un milieu de culture contenant hVEGF pour différenciation (comme défini dans l'article de Le Ricousse-Roussanne S. *et al., Cardiosvasc. Res., 2004; 62*: *176-184*). Après 15 jours de culture, on recueille un mélange cellulaire enrichi en EPC-EphB4.
***Exemple 2***

### Obtention de EPC-EphB4

(***A***) A partir du mélange cellulaire obtenu à l'issue de l'exemple 1(A), on isole et purifie les cellules CD34⁺ des cellules non adhérentes par une technique de séparation immunomagnétique standard, notamment au moyen du matériel "CD34 isolation Kit" (commercialisé par la société dite MILTENYI BIOTECH, Paris France), qui comporte un anticorps monoclonal anti-CD34. L'analyse des cellules, ainsi obtenues, par cytométrie de flux et en utilisant un anticorps monoclonal anti-CD34 (de préférence différent du précédent) couplé au FITC, montre que 75% (± 5,6%) d'entre elles possèdent le marqueur CD34.
***(B)*** Le mélange cellulaire, ainsi obtenu, qui contient 1,5 x 10⁶ à 3,5 x 10⁶ cellules CD34⁺, peut être placé dans les puits d'une plaque à 6 puits revêtus d'une matrice contenant fibronectine, laminine, sulfate sodique d'héparane, et collagène de type I et IV (produits commercialisés par la société dite SIGMA-ALDRICH précitée) et dans un milieu de culture contenant hVEGF, bFGF et IGF1 (produits commercialisés par la société dite R&D SYSTEMS INC., Oxford, Royaume-Uni). Après 15 jours de culture, on recueille un mélange cellulaire enrichi en EPC-EphB4.

### Exemple 3

### Obtention de EPC-EphB4-éphrine B2-Fc

Après obtention du mélange cellulaire selon l'exemple 1 (B), qui contient des cellules EPCs pourvues du marqueur EphB4, on le traite avec 3µg/ml de protéine de fusion Ephrine-B2-Fc, EphB4-Fc ou CD6-Fc (CD6-Fc intervenant en tant que contrôle négatif pour la démonstration de l'effet observé par l'activation des EPCs par éphrine-B2) pendant une durée d'incubation de 30 minutes à 37 °C. On écarte par rinçage chaque protéine de fusion non lié (au moins deux rinçages sont effectués ici).

On obtient trois mélanges cellulaires dont l'un contient le matériau cellulaire selon l'invention de structure EPC-EphB4-éphrine-B2-Fc, le second contient le matériau cellulaire résultant de l'activation de EPC par la protéine de fusion EphB4-Fc, et le troisième contient le matériau cellulaire résultant de l'activation de EPC par la protéine de fusion CD6-Fc.

### Exemple 4

### Obtention de EPC-EphB4-éphrine-B2-Fc

On procède comme indiqué à l'exemple 3 pour l'obtention de cellules de structure EPC-EphB4-éphrine-B2-Fc, avec la différence que le mélange cellulaire de départ est celui obtenu à l'issue de l'exemple 2(B). On obtient les cellules activées annoncées.

### Protocole des essais

A l'instant T = 0, on procède à la ligature de l'artère fémorale droite de souris mâles Nude âgées de 7 semaines (un lot de 6 animaux par essai et par produit à tester, y compris les témoins PBS, EPCs non activées et HUVEC) pour induire une ischémie. A l'instant T = + 4,5 h, on met en oeuvre l'incubation selon l'exemple 3 pour obtenir le matériau cellulaire de structure EPC-EphB4-éphrine-B2-Fc selon l'invention et les deux autres matériaux cellulaires de comparaison. Ensuite, à l'instant T = + 5 h, on injecte par voie intraveineuse au niveau du sinus rétro-orbital chacun des trois mélanges cellulaires obtenus à l'exemple 3 (10⁶ cellules/souris). A l'instant T = + 12 j, les souris sont sacrifiées et les muscles gastrocnemius de la patte ischémiée et de la patte non ischémiée sont prélevés. On détermine :
- le score angiographique ;
- la densité capillaire (i. e. le nombre de capillaires/mm²) ; et
- le flux sanguin cutané.

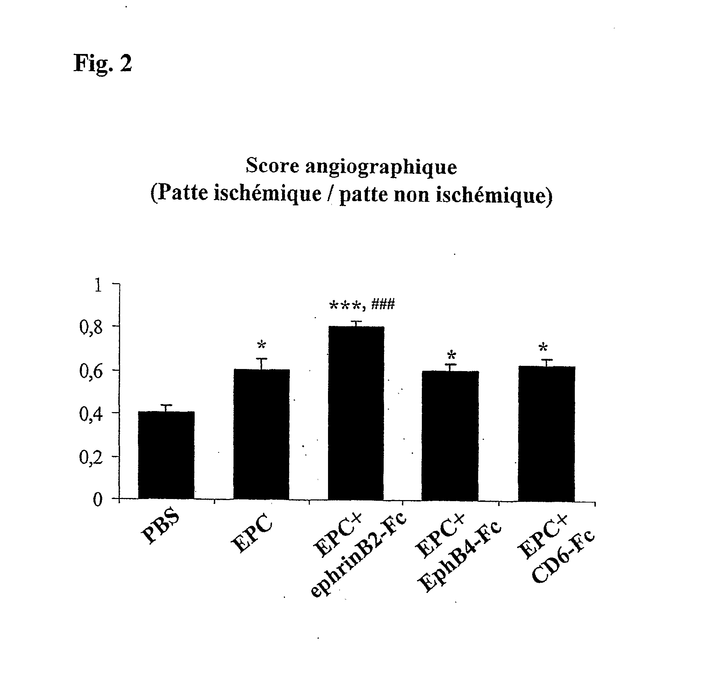

Dans les figures 2-4, les valeurs numériques obtenues sont présentées sous la forme : moyenne ± SEM.

### Essai 1

### Score angiographique

Le score angiographique (i . e. densité des vaisseaux) a été déterminé par micro-angiographie. Plus précisément, la densité des vaisseaux dans le membre ischémié par rapport au membre non ischémié est mesurée par une micro-angiographie de haute définition (voir modalités opératoires dans l'article de Sivestre J. S et al., Cir. Res., 2001; 89: 259-264*).* Les résultats obtenus, présentés sous forme de rapport (patte ischémiée/patte non ischémiée), sont consignés graphiquement à la figure 2.

On observe que l'injection des EPCs non activées ou activées par la protéine de fusion EphB4-Fc ou CD6-Fc augmentet légèrement, et à des niveaux comparables, la densité des vaisseaux par rapport au groupe contrôle ayant reçu le PBS. Lorsque les cellules EPCs sont activées par la protéine de fusion éphrine-B2-Fc, on constate que l'augmentation de la densité des vaisseaux est de 25,5% plus importante que celle observée après injection des EPCs non activées, soit une augmentation du score angiographique de 1,34 fois.

### Essai 2

### Densité capillaire (nombre de capillaires/mm²)

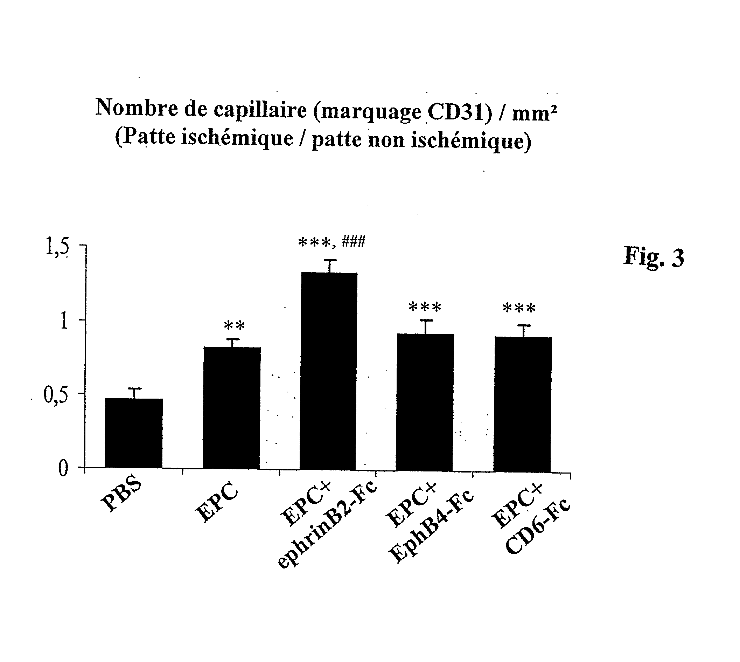

La densité capillaire du muscle ischémié a été étudiée par marquage de coupes du muscle gastrocnemius au moyen d'un anticorps dirigé contre le marqueur CD31, qui est spécifique des cellules endothéliales, par rapport aux coupes du même muscle du membre non ischémié. Les résultats obtenus, présentés sous forme de rapport (patte ischémiée/patte non ischémiée), sont consignés graphiquement à la figure 3.

On observe que la densité capillaire est de 36,7% plus importante lorsque les souris ont été traitées par injection avec les EPCs activées avec la protéine de fusion éphrine-B2-Fc selon l'invention, par rapport aux EPCs non activées, soit une augmentation de la densité capillaire de 1,57 fois.

### Essai 3

### Flux sanguin cutané

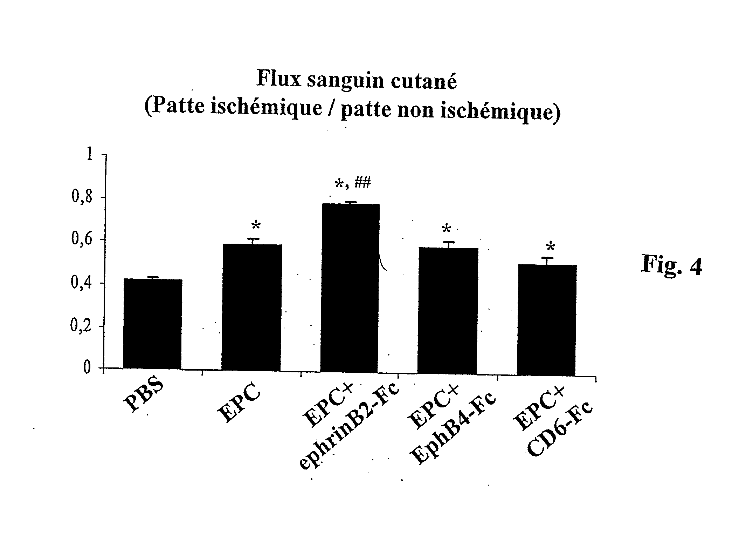

Une évaluation quantitative du flux sanguin, exprimée par le ratio du flux sanguin membre ischémié/membre non ischémié, a aussi été réalisée afin de vérifier que la variation du nombre de vaisseaux correspond à une adaptation fonctionnelle et donc à une variation de la perfusion du membre ischémié. Les résultats obtenus sont consignés graphiquement à la figure 4.

On observe que l'injection des EPCs activées selon l'invention par la protéine de fusion éphrine-B2-Fc augmente le ratio du flux sanguin du membre ischémié à celui du membre non ischémié de 1,37 fois (augmentation de 27,1 %).

### Exemple 5

### Rôle du marqueur EphB4

Dans ce dernier exemple, le rôle du marqueur EphB4 a été démontré grâce à des préparations cellulaires dans lesquelles la synthèse protéinique du marqueur EphB4 a été inhibée.

Pour cela, on a fait appel à des « ARN interférences » ou « siRNA », en langue anglaise, capables notamment, de dégrader spécifiquement les ARN messagers codant pour un gène donné, et en particulier ici pour le gène exprimant le marqueur EphB4. L'action spécifique de ces ARN interférences est dénommée transfection.

Ces préparations cellulaires ont été administrées à des souris mâles Nude selon un protocole d'essais analogue au protocole d'essais précité.

En premier lieu, des cellules endothéliales progénitrices, ou EPCs sont cultivées jusqu'à 80 % de confluence. Des solutions de siRNA EphB4, c'est-à-dire contenant l'ARN interférence, capable d'inhiber la synthèse protéinique du marqueur EphB4 sont dilués dans un milieu M199 ne contenant ni antibiotiques, ni sérum et incubées pendant cinq minutes à température ambiante. Par ailleurs, une solution de « siRNA contrôle » ne correspondant à aucun gène particulier, est diluée dans les mêmes conditions. Cette dernière solution diluée qui n'a donc aucun effet biologique sur l'expression du marqueur EphB4 va simplement permettre de vérifier que les ARN interférences n'ont pas d'activité propre indépendamment de leur rôle d'inhibiteur.

Par ailleurs, un agent de transfection, le Dharmafect2 (Dharmacon, Perbio) est préparé dans les mêmes conditions que les solutions précitées, puis mélangé à ces solutions, lesquelles sont alors incubées ensuite.

Ces solutions incubées sont alors mises en contact avec les cellules endothéliales progénitrices EPCs de manière à, dans une première préparation cellulaire, provoquer l'inhibition de la synthèse protéinique du marqueur EphB4, et dans une deuxième préparation cellulaire ou l'expression du gène codant pour le marqueur EphB4 n'est pas inhibée, à constituer un témoin.

Ces deux préparations cellulaires seront à leur tour respectivement divisées en deux et l'une d'entre elles sera stimulée avec la protéine de fusion éphrine-B2-Fc avant d'être injectée par voie intraveineuse aux souris ayant subi une ligature de l'artère fémorale droite. De la sorte, quatre préparations cellulaires différentes seront administrées aux souris.

On trouvera dans le tableau I suivant, une synthèse des résultats de cet exemple 5.

**Tableau I**

| N° | **PBS** | **1** | **2** | **3** | **4** |
|---|---|---|---|---|---|
| **Score Angiographie** | 100 | 161,5 +/- 10,7 | 218,8 +/- 12,8 | 153,5 +/- 2,9 | 158,9 +/- 12,5 |
| **Flux Sanguin** | 100 | 144,5 +/- 4,5 | 193,5 +/- 4,7 | 151,1 +/- 8 | 138,5 +/- 14,1 |
| **Densité Capilaire** | 100 | 147,4 +/- 10,7 | 201,7 +/- 12,8 | 142,6 +/- 11 | 140,8 +/-11,3 |

| | | | | | |
|---|---|---|---|---|---|
| 1 : EPCs transfectées avec le siRNA contrôle et non stimulées avant injection ; 2 : EPCs transfectées avec le siRNA contrôle et stimulées avec éphrine-B2-Fc avant injection 3 : EPCs transfectées avec le siRNA EphB4 et non stimulées avant injection ; 4 : EPCs transfectées avec le siRNA EphB4 et stimulées avec éphrine-B2-Fc avant injection. | | | | | |

On notera là encore, que les mesures du score angiographique, du flux sanguin et de la densité capillaire pour les quatre préparations cellulaires administrées, évoluent de manière sensiblement parallèle.

Par ailleurs, il apparaît immédiatement que le résultat des mesures des préparations cellulaires n° 1, 3 et 4 sont sensiblement identiques alors que le résultat de la préparation cellulaire n°2 est supérieur de près de 40 %.

L'hypothèse étant bien évidemment que l'activité pro-angiogénique est favorisée par l'association du marqueur EphB4 et du matériau protéinique éphrine-B2-Fc, on constate à la lecture du tableau I, en comparant les résultats des préparations cellulaires n° 1 et 4, que l'effet des cellules endothéliales progénitrices incorporant le « siRNA contrôle » mais non associées au matériau protéinique éphrine-B2-Fc est sensiblement équivalent à l'effet des cellules endothéliales progénitrices dont l'expression du marqueur EphB4 est inhibée mais qui elles sont associées au matériau protéinique éphrine-B2-Fc. Ainsi, il est montré que les ARN interférences n'ont pas d'activité propre indépendamment de leur rôle d'inhibiteur et que les cellules endothéliales progénitrices EPCs exprimant le marqueur EphB4 n'ont pas plus d'activité que les cellules endothéliales progénitrices EPCs n'exprimant pas le marqueur et qui sont associés au matériau protéinique éphrine-B2-Fc.

Par ailleurs, en comparant les résultats des mesures des préparations cellulaires n° 1 et 3, il est montré que les cellules endothéliales progénitrices EPCs seules, exprimant le marqueur EphB4 ou ne l'exprimant pas ont une activité comparable.

En outre, et c'est là l'essentiel, il est bien montré au vu des résultats de la préparation cellulaire n°2 et en comparaison de l'une quelconque des autres préparations, que l'association spécifique du marqueur EphB4 et du matériau protéinique éphrine-B2-Fc présente une activité pro-angiogénique importante.

**Tableau II**

| Spécificités pour quelques couples Eph/éphrine | |
|---|---|
| Récepteurs Eph | Ligands éphrine (par affinité décroissante) |
| EphA1 | éphrine-A1 |
| EphA2 | éphrine-A3, -A1, -A5, -A4 |
| EphA3 | éphrine-A5, -A2, -A3, -A1 |
| EphA4 | éphrine-A5, -A1, -A3, -A2, -B2, -B3 |
| EphA5 | éphrine-A5, -A1, -A2, -A3, -A4 |
| EphA6 | éphrine-A2, -A1, -A3, -A4, -A5 |
| EphA7 | éphrine-A2, -A3, -A1 |
| EphA8 | éphrine-A5, -A3, -A2 |
| EphB1 | éphrine-B2, -B1, -A3 |
| EphB2 | éphrine-B1, -B2, -B3 |
| EphB3 | éphrine-B1, -B2, -B3 |
| EphB4 | éphrine-B2, -B1 |

## Revendications

1. Système proangiogénique du type marqueur cellulaire/ligand spécifique, ledit système, dans lequel le marqueur cellulaire est présent sur la membrane extérieure de la cellule, étant **caractérisé en ce qu'**il comprend :
(a) un précurseur de cellule endothéliale (EPC) comportant un marqueur cellulaire choisi parmi l'ensemble constitué par les marqueurs cellulaire EphB, notamment EphB4 ou EphB1, et
(b) un matériau protéinique de structure :
L-K (I)
qui est constitué d'un ligand (L) choisi parmi les ligands éphrine-B ou un fragment peptidique de ceux-ci ayant la même activité biologique, et est associé ou fusionné avec une protéine de liaison (K) choisie parmi des fragments Fc d'anticorps A,
ledit précurseur de cellule endothéliale (a) et ledit matériau protéinique (b) sont conditionnés séparément l'un de l'autre,
et **en ce que** l'incubation dudit précurseur de cellule endothéliale (a) et dudit matériau protéinique (b) fournit un matériau cellulaire de structure :
EPC-EphB-L-K (II)
pour stimuler l'angiogenèse.

2. Système suivant la revendication 1, **caractérisé en ce que** ledit marqueur EphB est sous la forme d'une séquence d'aminoacides.

3. Système suivant la revendication 1 ou 2, **caractérisé en ce que** ledit marqueur est EphB4 ou EphB1, et **en ce que** ledit ligand est éphrine-B2.

4. Système suivant la revendication 1 ou 2, **caractérisé en ce que** ledit marqueur et ledit ligand sont respectivement EphB4 et éphrine-B 1.

5. Système suivant l'une quelconque des revendications 1 à 4, **caractérisé en ce que** les EPCs sont obtenues à partir de cellules mononucléées choisies parmi les CD34⁺ et CD133⁺, ou de cellules exprimant CD34 ou CD133, qui proviennent de la moelle osseuse, du sang périphérique ou mieux du sang de cordon ombilical.

6. Système suivant l'une quelconque des revendications 1 à 5, **caractérisé en ce que** les EPCs sont obtenues à partir de sang périphérique ou de sang de cordon ombilical.

7. Matériau cellulaire pour stimuler l'angiogenèse, **caractérisé en ce qu'**il a pour structure :
EPC-EphB-L-K (II)
où ledit ligand L choisi parmi les ligands éphrine-B ou un fragment peptidique de ceux-ci ayant la même activité biologique, est associé à ou fusionné avec une protéine de liaison K choisie parmi des fragments Fc d'anticorps A, ledit matériau cellulaire étant susceptible (i) de se présenter sous la forme d'une culture cellulaire purifiée ou sous la forme d'une culture cellulaire en association avec d'autres cellules précurseurs, notamment des cellules mononucléées, et (ii) d'être; le cas échéant, congelé.

8. Matériau cellulaire suivant la revendication 7, **caractérisé en ce qu'**il est obtenu par incubation
(a) d'un précurseur de cellule endothéliale (EPC) comportant un marqueur cellulaire choisi parmi l'ensemble constitué par les EphB, notamment EphB4 ou EphB 1, avec
(b) un matériau protéinique de structure :
L-K (I)
qui est constitué d'un ligand (L) spécifique dudit marqueur, et est associé ou fusionné avec une protéine de liaison (K), choisie parmi un fragment Fc d'anticorps A,
avant d'être amené à son site d'administration.

9. Matériau cellulaire suivant la revendication 7 ou 8, **caractérisé en ce que** ledit marqueur est EphB4 ou EphB1, et **en ce que** ledit ligand est éphrine-B2.

10. Procédé pour la préparation d'un matériau cellulaire selon l'une quelconque des revendications 7 à 9, ledit procédé étant **caractérisé en ce qu'**il comprend les étapes consistant à :
• faire appel à des EPCs exprimant sur leur membrane extérieure un marqueur de la famille des EphB, notamment EphB4 ou EphB1, et est choisi parmi les ligands éphrine-B ou un fragment peptidique de ceux-ci ayant la même activité biologique,
• mettre en contact *in vitro* lesdits EPCs avec un matériau protéinique L-K dans lequel : L est un ligand spécifique dudit marqueur et est choisi parmi les ligands Ephrine-B ou un fragment peptidique de ceux-ci ayant la même activité biologique ;et K est une protéine de liaison associée ou fusionnée à L choisie parmi des fragments Fc d'anticorps A.

11. Procédé suivant la revendication 10, **caractérisé en ce qu'**il comprend les étapes suivantes :
(1°) faire appel à des cellules mononucléées provenant de la moelle osseuse, du sang périphérique ou du sang de cordon ombilical, et isoler les cellules mononucléées CD34⁺ et/ou CD133⁺.
(2°) différencier lesdites cellules de l'étape précédente pour obtenir des EPCs pourvues du marqueur EphB, et
(3°) activer *in vitro* les EPCs, ainsi obtenues à l'étape (2°), par fixation d'éphrine-B sur EphB.

12. Procédé suivant la revendication 11, **caractérisé en ce que**, à l'étape (1°), les cellules mononucléées proviennent du sang périphérique ou du sang de cordon ombilical.

13. Procédé suivant l'une quelconque des revendications 10 à 12, **caractérisé en ce que** ledit ligand L est choisi parmi les ligands éphrine-B ou un fragment peptidique de ceux-ci ayant la même activité biologique et est associé à ou fusionné avec une protéine de liaison K pour fournir un matériau cellulaire de structure :
EPC-EphB-L-K (II)
où K est choisi parmi un fragment Fc d'anticorps A.

14. Procédé suivant l'une quelconque des revendications 10 à 13, **caractérisé en ce que** le marqueur est EphB4 ou EphB1, et **en ce que** ledit ligand L est éphrine-B2.

15. Médicament utilisable pour reconstituer les vaisseaux lésés, **caractérisé en ce qu'**il comprend, en association avec un excipient physiologiquement acceptable, une quantité thérapeutiquement acceptable d'un matériau cellulaire selon l'une quelconque des revendications 7 à 9, notamment en tant qu'ingrédient actif proangiogénique.

16. Composition médicamenteuse pour reconstituer les vaisseaux lésés, **caractérisée en ce qu'**elle comprend, une quantité thérapeutiquement acceptable des deux composants d'un système proangiogénique selon l'une quelconque des revendications 1 à 6, qui sont conditionnés séparément, chacun dans un excipient physiologiquement acceptable, les deux dits composants étant incubés avant administration pour donner un matériau cellulaire de structure II.

17. Utilisation d'un matériau cellulaire, ladite utilisation étant **caractérisée en ce que** l'on fait appel à un matériau cellulaire selon l'une quelconque des revendications 7 à 9, en tant qu'ingrédient actif proangiogénique, en association avec un excipient physiologiquement acceptable, pour la préparation d'une composition pour usage thérapeutique dans le traitement des insuffisances vasculaires, notamment dans la revascularisation des tissus ischémiés, cardiaques, cérébraux ou périphériques.

18. Utilisation suivant la revendication 17, **caractérisée en ce que** ladite composition est destinée au traitement de l'artérite, de l'insuffisance coronarienne, de l'insuffisance cardiaque ou de l'insuffisance cérébrale.

## Patentansprüche

1. Proangiogenes System vom Typ Zellmarker/spezifischer Ligand, wobei das System, in dem der Zellmarker auf der äußeren Membran der Zelle vorhanden ist, **dadurch gekennzeichnet ist, dass** es umfasst:
(a) einen Endothelzellen-Vorläufer (EPC), enthaltend einen Zellmarker, ausgewählt aus der Gruppe, gebildet von EphB-Zellmarkern, insbesondere EphB4 oder EphB1, und
(b) ein Proteinmaterial mit der Struktur:
L-K (I)
welches von einen Liganden (L), ausgewählt aus den Ephrin-B-Liganden oder einem Peptidfragment davon mit der gleichen biologischen Aktivität, gebildet ist und mit einem Bindungsprotein (K), ausgewählt aus Fc-Fragmenten eines A-Antikörpers, assoziiert oder daran gebunden ist,
wobei der Endothelzellen-Vorläufer (a) und das Proteinmaterial (b) voneinander getrennt bereitgestellt sind,
und dadurch, dass die Inkubation des Endothelzellen-Vorläufers (a) und des Proteinmaterials (b) ein Zellmaterial mit der Struktur:
EPC-EphB-L-K (II)
zum Stimulieren der Angiogenese bereitstellt.

2. System nach Anspruch 1, **dadurch gekennzeichnet, dass** der Marker EphB in der Form einer Aminosäuresequenz vorliegt.

3. System nach Anspruch 1 oder 2, **dadurch gekennzeichnet, dass** der Marker EphB4 oder EphB1 ist, und dadurch, dass der Ligand Ephrin-B2 ist.

4. System nach Anspruch 1 oder 2, **dadurch gekennzeichnet, dass** der Marker und der Ligand jeweils EphB4 und Ephrin-B1 sind.

5. System nach einem der Ansprüche 1 bis 4, **dadurch gekennzeichnet, dass** die EPCs aus einkernigen Zellen, ausgewählt aus CD34⁺ und CD133⁺ oder aus Zellen, welche CD34 oder CD133 exprimieren, welche aus Knochenmark, peripherem Blut oder vorzugsweise Nabelschnurblut entstammen, erhalten wurden.

6. System nach einem der Ansprüche 1 bis 5, **dadurch gekennzeichnet, dass** die EPCs aus peripherem Blut oder Nabelschnurblut erhalten wurden.

7. Zellmaterial zur Stimulierung der Angiogenese, **dadurch gekennzeichnet, dass** es die Struktur:
EPC-EphB-L-K (II)
aufweist,
wobei der Ligand L, ausgewählt aus den Ephrin-B-Liganden oder einem Peptidfragment davon mit der gleichen biologischen Aktivität, mit einem K-Bindungsprotein, ausgewählt aus Fc-Fragmenten eines A-Antikörpers, assoziiert oder daran gebunden ist, wobei das Zellmaterial dazu geeignet ist (i) in Form einer aufgereinigten Zellkultur oder in Form einer Zellkultur zusammen mit anderen Vorläuferzellen, insbesondere einkernigen Zellen, vorzuliegen und (ii) gegebenenfalls eingefroren zu werden.

8. Zellmaterial nach Anspruch 7, **dadurch gekennzeichnet, dass** es durch Inkubation
(a) eines Endothelzellen-Vorläufers (EPC), umfassend einen Zellmarker, ausgewählt aus der Gruppe, gebildet durch die EphB, insbesondere EphB4 oder EphB1, mit
(b) einem Proteinmaterial der Struktur:
L-K (I)
welches durch einen für den Marker spezifischen Liganden (L) gebildet ist und mit einem Bindungsprotein (K), ausgewählt aus einem Fc-Fragment eines A-Antikörpers, assoziiert oder daran gebunden ist,
erhalten wird,
bevor es seinem Verabreichungsort zugeführt wird.

9. Zellmaterial nach Anspruch 7 oder 8, **dadurch gekennzeichnet, dass** der Marker EphB4 oder EphB1 ist, und dadurch, dass der Ligand Ephrin-B2 ist.

10. Verfahren zur Herstellung eines Zellmaterials nach einem der Ansprüche 7 bis 9, wobei das Verfahren **dadurch gekennzeichnet ist, dass** es die Schritte, bestehend aus:
• Bereitstellen von EPCs, welche auf ihrer äußeren Membran einen Marker der EphB-Familie, insbesondere EphB4 oder EphB1, exprimieren, welcher aus Ephrin-B-Liganden oder einem Peptidfragment davon mit der gleichen biologischen Aktivität ausgewählt ist,
• Inkontaktbringen *in vitro* der EPCs mit einem Proteinmaterial L-K, in dem: L ein spezifischer Ligand des Markers ist und aus den Ephrin-B-Liganden oder einem Peptidfragment davon mit der gleichen biologischen Aktivität ausgewählt ist; und K ein Bindungsprotein, assoziiert mit oder gebunden an L, ausgewählt aus Fc-Fragmenten eines A-Antikörpers, ist,
umfasst.

11. Verfahren nach Anspruch 10, **dadurch gekennzeichnet, dass** es die folgenden Schritte umfasst:
(1) Bereitstellen einkerniger Zellen, entstammend aus Knochenmark, peripherem Blut oder Nabelschnurblut, und Isolieren der einkernigen Zellen CD34⁺ und/oder CD133⁺,
(2) Differenzieren der Zellen aus dem vorhergehenden Schritt zum Erhalten von EPCs, welche EphB-Marker aufweisen, und
(3) Aktivieren *in vitro* der so aus Schritt (2) erhaltenen EPCs zur Fixierung von Ephrin-B auf EphB.

12. Verfahren nach Anspruch 11, **dadurch gekennzeichnet, dass** im Schritt (1) die einkernigen Zellen aus peripherem Blut oder Nabelschnurblut entstammen.

13. Verfahren nach einem der Ansprüche 10 bis 12, **dadurch gekennzeichnet, dass** der Ligand L aus Ephrin-B-Liganden oder einem Peptidfragment davon mit der gleichen biologischen Aktivität ausgewählt ist und mit einem Bindungsprotein K assoziiert oder daran gebunden ist, zum Bereitstellen eines Zellmaterials der Struktur:
EPC-EphB-L-K (II)
wobei K aus einem Fc-Fragment eines A-Antikörpers ausgewählt ist.

14. Verfahren nach einem der Ansprüche 10 bis 13, **dadurch gekennzeichnet, dass** der Marker EphB4 oder EphB1 ist und dadurch, dass der Ligand L Ephrin-B2 ist.

15. Medikament, verwendbar zur Neubildung geschädigter Gefäße, **dadurch gekennzeichnet, dass** es, zusammen mit einem physiologisch akzeptablen Trägerstoff, eine therapeutisch akzeptable Menge eines Zellmaterials nach einem der Ansprüche 7 bis 9, insbesondere als proangiogen aktiven Inhaltsstoff, umfasst.

16. Medikamentöse Zusammensetzung zur Neubildung geschädigter Gefäße, **dadurch gekennzeichnet, dass** sie eine therapeutisch akzeptable Menge zweier Bestandteile eines proangiogenen Systems nach einem der Ansprüche 1 bis 6, welche getrennt bereitgestellt sind, jeder in einem physiologisch akzeptablen Trägerstoff, umfasst, wobei die beiden Bestandteile vor Verabreichung inkubiert werden, um ein Zellmaterial der Struktur II zu ergeben.

17. Verwendung eines Zellmaterials, wobei die Verwendung **gekennzeichnet ist durch** das Bereitstellen eines Zellmaterials nach einem der Ansprüche 7 bis 9 als proangiogen aktiven Inhaltsstoff, zusammen mit einem physiologisch akzeptablen Trägerstoff, zur Herstellung einer Zusammensetzung zur therapeutischen Verwendung für die Behandlung von Gefäßinsuffizienzen, insbesondere für die Revaskularisation von ischemischen, kardialen, zerebralen oder peripheren Geweben.

18. Verwendung nach Anspruch 17, **dadurch gekennzeichnet, dass** die Zusammensetzung für die Behandlung von Arteriitis, Koronarinsuffizienz, Kardialinsuffizienz oder Zerebralinsuffizienz bestimmt ist.

## Claims

1. Cell marker/specific ligand proangiogenic system, said system, in which the cell marker is present on the outer membrane of the cell, being **characterised in that** it comprises:
(a) an endothelial cell precursor (EPC) comprising a cell marker selected from the group consisting of the EphB cell markers, in particular EphB4 or EphB1, and
(b) a protein material of structure:
L-K (I)
which consists of a ligand (L) selected from the ephrin-B ligands or a peptide fragment thereof having the same biological activity, and is associated or fused with a binding protein (K) selected from A antibody Fc fragments,
said endothelial cell precursor (a) and said protein material (b) are packaged separately from one another,
and **in that** incubating said endothelial cell precursor (a) and said protein material (b) provides a cell material of structure:
EPC-EphB-L-K (II)
for stimulating angiogenesis.

2. System according to claim 1, **characterised in that** said EphB marker is in the form of an amino acid sequence.

3. System according to either claim 1 or claim 2, **characterised in that** said marker is EphB4 or EphB1 and **in that** said ligand is ephrin-B2.

4. System according to either claim 1 or claim 2, **characterised in that** said marker and said ligand are EphB4 and ephrin-B1 respectively.

5. System according to any of claims 1 to 4, **characterised in that** the EPCs are obtained from mononuclear cells selected from CD34⁺ and CD133⁺, or from cells expressing CD34 or CD133, which originate from the bone marrow, from peripheral blood or preferably from umbilical cord blood.

6. System according to any of claims 1 to 5, **characterised in that** the EPCs are obtained from peripheral blood or from umbilical cord blood.

7. Cell material for stimulating angiogenesis, **characterised in that** it has the structure:
EPC-EphB-L-K (II)
where said ligand L selected from the ephrin-B ligands or a peptide fragment thereof having the same biological activity is associated with or fused with a binding protein K selected from A antibody Fc fragments, said cell material possibly being (i) in the form of a purified cell culture or in the form of a cell culture in combination with other precursor cells, in particular mononuclear cells, and (ii), where appropriate, frozen.

8. Cell material according to claim 7, **characterised in that** it is obtained by incubating
(a) an endothelial cell precursor (EPC) comprising a cell marker selected from the group consisting of the EphBs, in particular EphB4 or EphB1, with
(b) a protein material of structure:
L-K (I)
which consists of a ligand (L) specific to said marker, and is associated or fused with a binding protein (K) selected from an A antibody Fc fragment,
before being brought to its site of administration.

9. Cell material according to either claim 7 or claim 8, **characterised in that** said marker is EphB4 or EphB1, and **in that** said ligand is ephrin-B2.

10. Process for preparing a cell material according to any of claims 7 to 9, said process being **characterised in that** it comprises the steps of:
• making use of EPCs expressing a marker of the EphB family, in particular EphB4 or EphB1, on their outer membrane, and is selected from the ephrin-B ligands or a peptide fragment thereof having the same biological activity,
• bringing said EPCs into contact, *in vitro,* with a protein material L-K in which L is a ligand specific to said marker and is selected from the ephrin-B ligands or a peptide fragment thereof having the same biological activity and K is a binding protein associated or fused with L selected from the A antibody Fc fragments.

11. Process according to claim 10, **characterised in that** it comprises the following steps:
(1°) making use of mononuclear cells originating from the bone marrow, from peripheral blood or from umbilical cord blood, and isolating the CD34⁺ and/or CD133⁺ mononuclear cells,
(2°) differentiating said cells of the preceding step in order to obtain EPCs having the EphB marker, and
(3°) activating the EPCs thus obtained in step (2°), *in vitro,* by binding ephrin-B to EphB.

12. Process according to claim 11, **characterised in that**, in step (1 °), the mononuclear cells originate from peripheral blood or from umbilical cord blood.

13. Process according to any of claims 10 to 12, **characterised in that** said ligand L is selected from the ephrin-B ligands or a peptide fragment thereof having the same biological activity and is associated with or fused with a binding protein K in order to provide a cell material of structure:
EPC-EphB-L-K (II)
where K is selected from an A antibody Fc fragment.

14. Process according to any of claims 10 to 13, **characterised in that** the marker is EphB4 or EphB1 and **in that** said ligand L is ephrin-B2.

15. Drug which can be used for reconstituting damaged vessels, **characterised in that** it comprises, in combination with a physiologically acceptable excipient, a therapeutically acceptable amount of a cell material according to any of claims 7 to 9, in particular as a proangiogenic active ingredient.

16. Medicinal composition for reconstituting damaged vessels, **characterised in that** it comprises a therapeutically acceptable amount of the two components of a proangiogenic system according to any of claims 1 to 6, which are packaged separately, each in a physiologically acceptable excipient, said two components being incubated before administration so as to give a cell material of structure II.

17. Use of a cell material, said use being **characterised in that** use is made of a cell material according to any of claims 7 to 9, as a proangiogenic active ingredient, in combination with a physiologically acceptable excipient, for the preparation of a composition for therapeutic use in the treatment of vascular insufficiencies, in particular in the revascularisation of ischemic, cardiac, cerebral or peripheral tissues.

18. Use according to claim 17, **characterised in that** said composition is intended to treat arteritis, coronary insufficiency, cardiac insufficiency or cerebral insufficiency.
